# EUROPÄISCHE PATENTSCHRIFT

(11) **EP 1 356 194 B1**
(45) Veröffentlichungstag und Bekanntmachung des Hinweises auf die Patenterteilung: **11.08.2004**
(21) Anmeldenummer: 02701205.3
(22) Anmeldetag: 18.01.2002
(51) Int. Cl.: F01N 3/20, B01D 53/94, G01N 27/06, B01D 53/90

(54) **VORRICHTUNG ZUR DOSIERUNG EINER ENZYMFREIEN HARNSTOFFLÖSUNG MIT SENSOREINHEIT ZUR KONTROLLE PHYSIKALISCHER ZUSTANDSGRÖSSEN DER HARNSTOFFLÖSUNG**
DEVICE FOR METERING A UREA SOLUTION DEVOID OF ENZYMES, COMPRISING A SENSOR UNIT FOR CONTROLLING PHYSICAL CONDITION VARIABLES OF SAID UREA SOLUTION
DISPOSITIF DE DOSAGE D'UNE COMPOSITION D'UREE DEPOURVUE D'ENZYMES, AVEC UNE UNITE DE DETECTION PERMETTANT LA VERIFICATION DE GRANDEURS PHYSIQUES D'ETAT DE LA COMPOSITION D'UREE

(30) Priorität: 19.01.2001 DE 10102237
(43) Veröffentlichungstag der Anmeldung: 29.10.2003
(73) Patentinhaber: ROBERT BOSCH GMBH, 70442 Stuttgart (DE)
(72) Erfinder: RIPPER, Wolfgang, 70327 Stuttgart (DE); BERGER, Joachim, 73650 Winterbach (DE); MAHR, Bernd, 73207 Plochingen (DE); BRINZ, Thomas, 73266 Bissingen unter der Teck (DE)
(86) Internationale Anmeldenummer: PCT/DE2002/000148
(87) Internationale Veröffentlichungsnummer: WO 2002/057603

(56) Entgegenhaltungen:
- EP-A- 0 905 356
- EP-A- 0 928 884
- WO-A-01/14045
- WO-A-99/30810

## Beschreibung

Die Erfindung betrifft eine Vorrichtung zur Dosierung einer Harnstofflösung nach dem Oberbegriff des Anspruchs 1.

### Stand der Technik

Zur Reduktion von Stickoxiden in dem Abgas von Kraftfahrzeugen wird während einer katalytischen Reduktion bislang bereits Harnstofflösung in das Abgas eingesprüht. Durch eine chemische Reaktion an einem Hydrolysekatalysator zersetzt sich der Harnstoff in Kohlendioxid und Ammoniak. Ammoniak wiederum reagiert sehr selektiv mit Stickoxiden unter der Bildung von Stickstoff und Wasser, so dass das Abgas von Stickstoffoxiden gereinigt wird.

Für die zuverlässige Reduktion von Stickoxiden mit einer Harnstofflösung sind verschiedene Parameter, insbesondere die Konzentration des Harnstoffs in der wässrigen Lösung von Bedeutung.

Bisher bekannte Sensoranwendungen zur Messung der Harnstoffkonzentration im Bereich der Medizin und der Biologie verwenden Urease, mit der enzymatisch der Harnstoff selektiv unter Bildung von Ammoniak aufgespalten wird.

Sensoren detektieren anschließend die Einflussnahme des Ammoniaks auf den pH-Wert der Lösung. Hierdurch können Rückschlüsse auf die Harnstoffkonzentration gezogen werden.

Nachteilig bei dieser Art der Konzentrationsmessung einer Harnstofflösung ist die Instabilität der Urease, insbesondere bei einer Umgebung, die sehr.unterschiedliche Temperaturen aufweisen kann. Derartige Temperaturschwankungen sind jedoch beim Einsatz in Kraftfahrzeugen vorgegeben, so dass die bisherigen Sensoren gemäß dem Stand der Technik für diese Anwendung nicht einsetzbar sind.

### Vorteile der Erfindung

Aufgabe der Erfindung ist es daher, eine Vorrichtung zur Dosierung von Harnstofflösungen vorzuschlagen, die auch unter schwierigen Bedingungen, beispielsweise innerhalb großer Temperaturintervalle zuverlässig zur Reduktion von Stickoxiden einsetzbar ist.

Diese Aufgabe wird ausgehend von einem Stand der Technik der einleitend genannten Art durch die kennzeichnenden Merkmale des Anspruchs 1 gelöst.

Durch die in den Unteransprüchen genannten Maßnahmen sind vorteilhafte. Ausführungen und Weiterbildungen der Erfindung möglich.

Dementsprechend zeichnet sich eine erfindungsgemäße Vorrichtung zur Dosierung von Harnstoff dadurch aus, dass eine Sensoreinheit zur Kontrolle einer physikalischen Zustandsgröße einer enzymfreien Harnstofflösung vorgesehen ist. Die Sensoreinheit umfasst hierbei vorzugsweise einen physikalischen Messwertaufnehmer.

Auf diese Weise ist eine Messung unmittelbar unter Zugrundelegung der physikalischen Eigenschaften des Harnstoffs in der Lösung ohne Zwischenschaltung einer enzymatischen Aufspaltung möglich. Die Messung unterliegt dementsprechend auch nicht den Instabilitäten, denen ein Enzym, wie z.B. Urease unterworfen ist.

In einer bestimmten Ausführungsform der Erfindung wird ein Messwertaufnehmer zur Erfassung einer oder mehrerer elektrischer Zustandsgrößen vorgesehen. Eine solche Zustandsgröße kann beispielsweise der pH-Wert, die Dielektrizitätskonstante und/oder der Leitwert der Lösung sein. Über die Messung dieser oder weiterer elektrischer Zustandsgrößen lassen sich Rückschlüsse auf die Beschaffenheit der Harnstofflösung, beispielsweise deren Konzentration ziehen. Die Messung dieser Zustandsgrößen ist dabei vergleichsweise unproblematisch und lässt sich insbesondere im Bereich großer Temperaturschwankungen durchführen.

Zur Erfassung der elektrischen Zustandsgröße werden vorzugsweise zwei Elektroden vorgesehen, die in die Harnstofflösung hineinragen. Durch Beaufschlagung der Elektroden mit einer elektrischen Gleich- und/oder Wechselspannung lassen sich die genannten elektrischen Zustandsgrößen wie der pH-Wert, die Dielektrizitätskonstante und/oder der Leitwert unmittelbar erfassen.

Zur Verbesserung der Empfindlichkeit des Messwertaufnehmers empfiehlt es sich hierbei, eine Oberflächen vergrößernde Struktur an den Elektroden vorzusehen. Eine solche Oberflächen vergrößernde Struktur kann beispielsweise durch eine kammförmige Ausbildung der Elektroden erzielt werden, die zusätzlich den Vorteil hat, dass zwei derart ausgebildete Elektroden ineinandergreifend angeordnet werden können, so dass bei vergleichsweise großer Oberfläche zugleich ein geringer Abstand der beiden Elektroden einstellbar ist. Durch die große Oberfläche, insbesondere in Verbindung mit dem geringen Abstand kann die Prüfspannung bzw. der Prüfstrom und somit die Steuer- und Auswerteeinheit für einen erfindungsgemäßen Messwertaufnehmer entsprechend klein dimensioniert werden. Gegebenenfalls kann zur gleichzeitigen Bestimmung mehrerer Zustandsgrößen eine separate Elektrode vorgesehen werden. Mittels einer solchen dritten Elektrode kann beispielsweise der pH-Wert bestimmt werden, während über die beiden vorgenannten Elektroden eine andere Zustandsgröße, beispielsweise die Dielektrizitätskonstante ermittelt wird.

In einer weiteren vorteilhaften Ausführungsform der Erfindung wird ein Messwertaufnehmer zur Erfassung einer oder mehrerer physikalisch-mechanischer Zustandsgrößen der Harnstofflösung vorgesehen. Eine solche physikalisch-mechanische Zustandsgröße kann beispielsweise in Form der Viskosität oder der Dichte vorliegen.

Derartige physikalisch-mechanische Zustandsgrößen können hierbei auf herkömmliche Weise, beispielsweise durch Wiegen der Lösung bzw. eines Teils der Lösung oder durch Messung des Auftriebs eines Verdrängungskörpers, usw. ermittelt werden. In einer besonders vorteilhaften Ausführungsform wird jedoch die physikalisch-mechanische Zustandsgröße durch einen dynamischen Sensor erfasst. So kann eine physikalisch-mechanische Zustandsgröße beispielsweise mit Hilfe eines Schwingungsgenerators gemessen werden. Das Verhalten der Harnstofflösung bei einer Anregung mit Hilfe mechanischer Schwingungen hängt signifikant von den zu erfassenden physikalisch-mechanischen Zustandsgrößen, beispielsweise der Dichte oder Viskosität ab. Dieses Verhalten kann in einer vorteilhaften Ausführungsform unmittelbar am Schwingungsgenerator selbst messtechnisch, z.B. durch Strommessung, Frequenzmessung, usw. erfasst werden.

Als Schwingungsgenerator wird vorzugsweise ein Schwingquarz verwendet. Jede andere bekannte oder künftige Möglichkeit zur Anregung mechanischer Schwingungen ist jedoch ebenfalls denkbar. So könnte beispielsweise ein Piezokristall ebenso eingesetzt werden, wie ein hochdrehender Motor mit einer Unwucht oder auch eine elektromagnetische Spule in Verbindung mit einer Membran nach dem Lautsprecherprinzip.

In einer besonders vorteilhaften Ausführungsform wird eine Sensoreinheit mit einem Messwertaufnehmer für eine elektrische Zustandsgröße sowie mit einem Messwertaufnehmer für eine physikalisch-mechanische Zustandsgröße vorgesehen. Die Messwerte beider Messwertaufnehmer werden hierbei in einer Auswerteeinheit zur Bestimmung der Konzentration des Harnstoffs in der Lösung verwendet. Durch die Auswertung zweier voneinander unabhängiger Zustandsgrößen ergibt sich die Möglichkeit einer genaueren bzw. selektiveren Bestimmung der Harnstoffkonzentration.

Weiterhin wird vorteilhafterweise eine erfindungsgemäße Vorrichtung mit einem Temperatursensor kombiniert. Da die zu erfassenden Zustandsgrößen unter Umständen eine deutliche Temperaturabhängigkeit aufweisen können, kann durch gleichzeitige Messung und Berücksichtigung der Temperatur in der Auswertung der erfassten Zustandsgröße, beispielsweise zur Bestimmung der Harnstoffkonzentration in der Lösung, eine Bereinigung von Fehlern durch Temperaturschwankungen vorgenommen werden.

Insbesondere in Verbindung mit einer Dosiervorrichtung für Harnstofflösung ist es weiterhin von Vorteil, zusätzlich einen Füllstandsensor zur Messung des Befüllungsgrades eines Vorratsbehälters für die Harnstofflösung vorzusehen. In einem besonders vorteilhaften Ausführungsbeispiel wird ein solcher Füllstandsensor unmittelbar mit einem erfindungsgemäßen Messwertaufnehmer zur Erfassung einer oder mehrerer physikalischer Zustandsgrößen kombiniert.

Da der erfindungsgemäße Messwertaufnehmer deutliche Unterschiede bei der Messung in Lösung gegenüber der Messung in der Gasphase zeigt, kann hierdurch ohne weiteres auch ein Füllstand gemessen werden. Hierzu sind wiederum unterschiedliche Ausgestaltungen des erfindungsgemäßen Messwertaufnehmers denkbar. So kann beispielsweise ein erfindungsgemäßer Messwertaufnehmer in einer bestimmten Füllhöhe angebracht werden und als Schwellwertsensor beim Passieren des Schwellwerts durch den Füllstand dienen. Für eine genauere Füllstandsmessung bei unterschiedlichen Füllhöhen können auch mehrere Sensoren auf unterschiedlicher Höhe angebracht werden. Ein solches Sensorsystem kann beispielsweise in einem sich über die entsprechende Höhe erstreckenden Sensorgehäuse oder aber auch an einer beispielsweise stabförmigen Sensorhalterung montiert sein.

Eine kontinuierliche Füllstandsmessung wäre ebenso denkbar, indem der erfindungsgemäße Messwertaufnehmer so ausgebildet wird, dass er sich über eine entsprechende Höhe erstreckt. Das Sensorsignal ist dabei vom Verhältnis der Sensorbereiche abhängig, die in der Gasphase bzw. in der flüssigen Lösung angeordnet sind. Diese Sensorbereiche wiederum variieren mit dem Füllstand, so dass hierdurch aus dem Sensorsignal Rückschlüsse über den Füllstand gezogen werden können.

### Ausführungsbeispiel

Ein Ausführungsbeispiel der Erfindung ist in der Zeichnung dargestellt und wird anhand der Figur näher erläutert.

Die einzige Figur zeigt einen schematischen Aufbau eines erfindungsgemäßen Messwertaufnehmers.

Die Sensoreinheit 1 ist auf einer Sensorplatte 2 aufgebracht. Eine kammförmige Elektrode 3 ist in zwei Bereiche 4, 5 unterteilt. In dem oberen Bereich 5 sind die einzelnen Zinken der Kammstruktur weiter voneinander beabstandet als in dem unteren Bereich 4.

In den oberen Bereich 5 greift eine weitere Elektrode 6 mit einer entsprechenden Kammstruktur ein. Die beiden Elektroden 5 und 6 erstrecken sich über einen großen-Bereich der Sensorplatte 2 und stellen einen Füllstandsensor dar. Dem unteren Bereich 4 der Elektrode 3 gegenüberliegend ist eine dritte Elektrode 7 angeordnet. Die Elektrode 7 entspricht mit ihrer Kammstruktur der feineren Kammstruktur des unteren Bereichs 4 der Elektrode 3, das heißt, die Zinken sind weniger weit voneinander beabstandet.

Die Elektrode 7 bildet zusammen mit dem unteren Bereich 4 der Elektrode 3 einen erfindungsgemäßen Messwertaufnehmer zur Messung einer elektrischen Zustandsgröße, z.B. der Leitfähigkeit, der Dielektrizitätskonstanten, usw..

Im unteren Bereich der Sensorplatte 2 sind die elektrischen Anschlüsse 8 für die Elektroden 3, 6, 7 angebracht. Diese elektrischen Anschlüsse 8 können auf nicht näher dargestellte Weise über einen Steckverbinder angeschlossen werden.

Unterhalb des unteren Bereichs 4 der Elektrode 3 bzw. unterhalb der Elektrode 7 ist ein Schwingquarz 9 als Schwingungsgenerator zur Erfassung einer physikalischmechanischen Zustandsgröße, z.B. der Viskosität oder der Dichte angebracht. Der Schwingquarz 9 ist ebenfalls über die Anschlüsse 8 kontaktiert.

Die Sensorplatte 2 kann in einer besonderen Ausführungsform wenigstens teilweise als Leiterplatte ausgebildet sein, auf der die Elektroden flächig in Form von Leiterbahnen realisiert sind. In einer anderen Bauform dient die Sensorplatte 2 hingegen als Montageplatte für montierbare Elektroden.

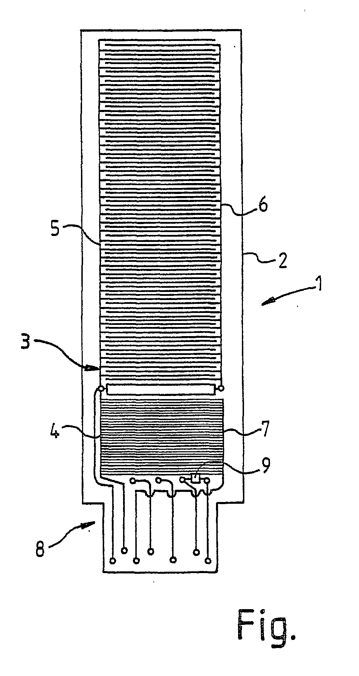

Mit Hilfe einer Sensoreinheit 1 gemäß der Figur 1 lässt sich sowohl eine oder mehrere elektrische Zustandsgrößen wie die Dielektrizitätskonstante, die Leitfähigkeit, der pH-Wert oder dergleichen als auch eine oder mehrere mechanischphysikalische Zustandsgrößen wie die Dichte oder Viskosität erfassen. Zugleich dient die Sensoreinheit 1 aufgrund der Ausdehnung des oberen Bereichs 5 der Elektrode 3 und der gegenüberliegenden Elektrode 6 als Füllstandssensor. Hierzu wird die Sensoreinheit 1 im Innern eines Behälters für eine Harnstofflösung angebracht, so dass die Elektroden 3 und 6 wenigstens teilweise in die Harnstofflösung eintauchen.

Mit Hilfe der erfindungsgemäßen Sensoreinheit 1 kann der Zustand einer Harnstofflösung auch bei widrigen Bedingungen, beispielsweise innerhalb großer Temperaturintervalle zuverlässig überwacht werden. Eine derartige Sensoreinheit 1 ist daher beispielsweise auch im Bereich der Abgasaufbereitung von Kraftfahrzeugen einsetzbar.

### Bezugszeichenliste:

- 1: Sensoreinheit
- 2: Sensorplatte
- 3: Elektrode
- 4: Bereich
- 5: Bereich
- 6: Elektrode
- 7: Elektrode
- 8: Anschlüsse
- 9: Schwingquarz

## Patentansprüche

1. Vorrichtung zur Dosierung einer Harnstofflösung, insbesondere zum Einsprühen der Harnstofflösung in den Abgasstrom einer Brennkraftmaschine, wobei eine Sensoreinheit zur Kontrolle einer oder mehrerer physikalischer Zustandsgrößen einer enzymfreien Harnstofflösung mit einem physikalischen Messwertaufnehmer (9) vorgesehen ist, **dadurch gekennzeichnet, dass** der Messwertaufnehmer (9) zur Erfassung der physikalisch-mechanischen Zustandsgröße Viskosität oder Dichte ausgebildet ist.

2. Vorrichtung nach Anspruch 1, **dadurch gekennzeichnet, dass** der Messwertaufnehmer (3, 6) zur Erfassung einer elektrischen Zustandsgröße ausgebildet ist.

3. Vorrichtung nach einem der vorgenannten Ansprüche, **dadurch gekennzeichnet, dass** der Messwertaufnehmer (3, 6, 7) zur Erfassung des pH-Werts, der die Dielektrizitätskonstante und/oder des Leitwerts der enzymfreien Harnstofflösung ausgebildet ist.

4. Vorrichtung nach einem der vorgenannten Ansprüche, **dadurch gekennzeichnet, dass** der Messwertaufnehmer (3, 6, 7) wenigstens zwei Elektroden umfasst.

5. Vorrichtung nach einem der vorgenannten Ansprüche, **dadurch gekennzeichnet, dass** wenigstens eine Elektrode (3, 6, 7) eine Oberflächen vergrößernde Struktur aufweist.

6. Vorrichtung nach einem der vorgenannten Ansprüche, **dadurch gekennzeichnet, dass** zwei Elektroden (3, 6) eine kammförmige, ineinandergreifende Struktur aufweisen.

7. Vorrichtung nach einem der vorgenannten Ansprüche, **dadurch gekennzeichnet, dass** wenigstens eine dritte Elektrode (7) zur Erfassung wenigstens einer zweiten elektrischen Zustandsgröße vorgesehen ist.

8. Vorrichtung nach einem der vorgenannten Ansprüche, **dadurch gekennzeichnet, dass** der Messwertaufnehmer (9) zur Messung der Viskosität und/oder der Dichte der enzymfreien Harnstofflösung ausgebildet ist.

9. Vorrichtung nach einem der vorgenannten Ansprüche, **dadurch gekennzeichnet, dass** ein Schwingungsgenerator (9) vorgesehen ist.

10. Vorrichtung nach einem der vorgenannten Ansprüche, **dadurch gekennzeichnet, dass** der Schwingungsgenerator einen Schwingquarz (9) und/oder einen Piezokristall umfasst.

11. Vorrichtung nach einem der vorgenannten Ansprüche, **dadurch gekennzeichnet, dass** eine Sensoreinheit (1) mit einem Messwertaufnehmer (3, 6, 7) für eine elektrische Zustandsgröße der Harnstofflösung und mit einem Messwertaufnehmer (9) für eine physikalisch-mechanische Zustandsgröße vorhanden ist, wobei eine Auswerteeinheit zur Bestimmung der Harnstoffkonzentration aus den beiden Messgrößen vorgesehen ist.

12. Vorrichtung nach einem der vorgenannten Ansprüche, **dadurch gekennzeichnet, dass** ein Temperatursensor vorgesehen ist.

13. Vorrichtung nach einem der vorgenannten Ansprüche, **dadurch gekennzeichnet, dass** ein Füllstandsensor für einen Vorratsbehälter vorgesehen ist.

14. Vorrichtung nach einem der vorgenannten Ansprüche, **dadurch gekennzeichnet, dass** der Füllstandsensor ein Messwertaufnehmer nach einem der vorgenannten Ansprüche ist.

15. Vorrichtung nach einem der vorgenannten Ansprüche, **dadurch gekennzeichnet, dass** mehrere Füllstandsensoren vorgesehen sind.

16. Brennkraftmaschine mit katalytischer Abgasbehandlung, **dadurch gekennzeichnet, dass** eine Vorrichtung zur Dosierung einer Harnstofflösung nach einem der vorgenannten Ansprüche vorhanden ist.

## Claims

1. Device for metering a urea solution, in particular for spraying the urea solution into the exhaust-gas stream from an internal combustion engine, in which there is a sensor unit for monitoring one or more physical state variables of an enzyme-free urea solution having a physical measured value pick-up (9) **characterized in that** the measured value pick-up (9) is designed to record the physical-mechanical state variable viscosity or density.

2. Device according to Claim 1, **characterized in that** the measured value pick-up (3, 6) is designed to record an electrical state variable.

3. Device according to one of the preceding claims, **characterized in that** the measured value pick-up (3, 6, 7) is designed to record the pH, the dielectric constant and/or the conductance of the enzyme-free urea solution.

4. Device according to one of the preceding claims, **characterized in that** the measured value pick-up (3, 6, 7) comprises at least two electrodes.

5. Device according to one of the preceding claims, **characterized in that** at least one electrode (3, 6, 7) has a structure which increases its surface area.

6. Device according to one of the preceding claims, **characterized in that** two electrodes (3, 6) have a comb-like, interdigitated structure.

7. Device according to one of the preceding claims, **characterized in that** there is at least a third electrode (7) for recording at least a second electrical state variable.

8. Device according to one of the preceding claims, **characterized in that** the measured value pick-up (9) is designed to measure the viscosity and/or density of the enzyme-free urea solution.

9. Device according to one of the preceding claims, **characterized in that** a vibration generator (9) is provided.

10. Device according to one of the preceding claims, **characterized in that** the vibration generator comprises a quartz resonator (9) and/or a piezocrystal.

11. Device according to one of the preceding claims, **characterized in that** there is a sensor unit (1) having. a measured value pick-up (3, 6, 7) for an electrical state variable of the urea solution and having a measured value pick-up (9) for a physical-mechanical state variable, an evaluation unit being provided for determining the urea concentration from the two measured variables.

12. Device according to one of the preceding claims, **characterized in that** there is a temperature sensor.

13. Device according to one of the preceding claims, **characterized in that** there is a level sensor for a storage tank.

14. Device according to one of the preceding claims, **characterized in that** the level sensor is a measured value pick-up as described in one of the preceding claims.

15. Device according to one of the preceding claims, **characterized in that** a plurality of level sensors are provided.

16. Internal combustion engine with catalytic exhaust-gas treatment, **characterized in that** there is a device for metering a urea solution as described in one of the preceding claims.

## Revendications

1. Dispositif de dosage d'une solution d'urée, notamment pour injecter la solution d'urée dans le flux de gaz d'échappement d'un moteur à combustion interne, une unité de capteur étant prévue avec un transducteur (9) physique, pour le contrôle d'une ou de plusieurs grandeurs d'état physiques d'une solution d'urée dépourvue d'enzymes
**caractérisé en ce que**
le transducteur (9) est configuré pour mesurer une grandeur d'état physico-mécanique telle que la viscosité ou la densité.

2. Dispositif selon la revendication 1,
**caractérisé en ce que**
le transducteur (3, 6) est configuré pour mesurer une grandeur d'état électrique.

3. Dispositif selon l'une quelconque des revendications précédentes,
**caractérisé en ce que**
le transducteur (3, 6, 7) est configuré pour mesurer la valeur pH, la constante diélectrique et/ou la conductance de la solution d'urée dépourvue d'enzymes.

4. Dispositif selon l'une quelconque des revendications précédentes,
**caractérisé en ce que**
le transducteur (3, 6, 7). comprend au moins deux électrodes.

5. Dispositif selon l'une quelconque des revendications précédentes,
**caractérisé en ce qu'**
au moins une électrode (3, 6, 7) présente une structure agrandissant des surfaces.

6. Dispositif selon l'une quelconque des revendications précédentes,
**caractérisé en ce que**
deux électrodes (3, 6) présentent une structure en forme de peigne s'engrenant.

7. Dispositif selon l'une quelconque des revendications précédentes,
**caractérisé en ce qu'**
au moins une troisième électrode (7) est prévue pour mesurer au moins une deuxième grandeur d'état électrique.

8. Dispositif selon l'une quelconque des revendications précédentes,
**caractérisé en ce que**
le transducteur (9) est configuré pour mesurer la viscosité et/ou la densité de la solution d'urée dépourvue d'enzymes.

9. Dispositif selon l'une quelconque des revendications précédentes,
**caractérisé en ce qu'**
un générateur d'oscillations (9) est prévu.

10. Dispositif selon l'une quelconque des revendications précédentes,
**caractérisé en ce que**
le générateur d'oscillations comprend un cristal oscillateur (9) et/ou un cristal piézoélectrique.

11. Dispositif selon l'une quelconque des revendications précédentes,
**caractérisé par**
la présence d'une unité de capteur (1) comprenant un transducteur (3, 6, 7) pour une grandeur d'état électrique de la solution d'urée et un transducteur (9) pour une grandeur d'état physico-mécanique, avec une unité d'exploitation prévue pour déterminer la concentration d'urée à partir des deux valeurs mesurées.

12. Dispositif selon l'une quelconque des revendications précédentes,
**caractérisé par**
un capteur de température.

13. Dispositif selon l'une quelconque des revendications précédentes,
**caractérisé par**
un capteur de niveau de remplissage prévu pour un réservoir.

14. Dispositif selon l'une quelconque des revendications précédentes,
**caractérisé en ce que**
le capteur de niveau de remplissage est un transducteur selon l'une quelconque des revendications précédentes.

15. Dispositif selon l'une quelconque des revendications précédentes,
**caractérisé par**
plusieurs capteurs de niveau de remplissage.

16. Moteur à combustion interne à traitement catalytique des gaz d'échappement,
**caractérisé par**
un dispositif de dosage d'une solution d'urée, selon l'une quelconque des revendications précédentes.
